# EUROPEAN PATENT APPLICATION

(11) **EP 3 466 472 A1**
(43) Date of publication of application: **10.04.2019**
(21) Application number: 18198396.6
(22) Date of filing: 03.10.2018
(51) Int. Cl.: A61M 16/08, A61M 15/00, A61M 16/14

(54) **IMPROVED ADAPTER FOR DRUG DELIVERY**

(30) Priority: 03.10.2017 CA 2981322
(71) Applicant: SMART RS INC., Ottawa, ON K1V 1T5 (CA)
(72) Inventor: FIORENZA, Francesco, Ottawa, Ontario KIV IT5 (CA)
(74) Representative: Urizar Anasagasti, Jesus Maria

(57) **Abstract**

An improved adapter for use in a ventilator circuit is provided allowing to deliver a greater amount of a drug within the ventilator circuit. The improved adapter has a ventilator section, a transition section and medication section that are all interconnected to one another form an improved adapter. The transition section is further comprised of a smooth inner converging wall having an optimal angle of convergence.

## Description

### FIELD

The present disclosure relates to adapters and more specifically to adapters for use in ventilator circuits.

### BACKGROUND

Depending on the diagnostic, patients on mechanical ventilation may require the treatment of inhaled medications. Currently, the standard therapy is with nebulizers or metered dose inhalers (MDI). With new inhaled medications being offered, such as the Respimat Soft Mist Inhaler there is no solution to offer clinicians where they can use existing components in their mechanical ventilator to maintain a closed ventilator circuit while incorporating the delivery of inhaled medications. The goal of maintaining a closed ventilator circuit is to prevent lung de-recruitment as well as infection control benefits. Current adapters used in order to deliver drugs within a ventilator circuit have low drug delivery rates.

The improved adapter of the present disclosure connects to existing components within a ventilator circuit and does not add any weight or dead space which can cause displacement of the endotracheal tube or effect ventilation to the patient.

The improved adapter of the present disclosure connects to a valved tee which is an existing product that can be incorporated into a mechanical ventilator circuit. The valved tee is designed to allow a clinician to insert a nebulizer, which opens the valve, and deliver medication, once the nebulizer is removed the internal valve seals keeping the circuit closed. The improved adapter of the present disclosure will allow a clinician to insert the adapter attached to the Respimat Soft Mist Inhaler from Boehringer Ingelhiem (but not limited to) into a valve tee to deliver a soft mist of medication. The removal of the improved adapter of the present disclosure will cause the valve in the valve tee to seal the ventilator circuit providing a closed circuit.

There is a need for an improved adapter allowing for a greater percentage of drug being delivered in a ventilator circuit.

### SUMMARY

The present disclosure provides an improved adapter for delivering a drug, the adapter comprising: a ventilator section for connecting to a ventilator circuit; a transition section connected to a first end of the ventilator section, the transition section having a smooth inner converging wall to increase deposition of the drug in the ventilator circuit; and, a medication section having one end connected to the transition section and an opposite end connected to a drug dispenser.

### BRIEF DESCRIPTION OF THE DRAWINGS

The embodiments of the present disclosure will now be described by reference to the following figures, in which identical reference numerals in different figures indicate identical elements and in which:
**FIGURE 1** is a perspective view of an improved adapter for the delivery of a drug in a ventilator circuit according to one embodiment of the present disclosure;
**FIGURE 2** is a top view of an improved adapter for the delivery of a drug in a ventilator circuit according to one embodiment of the present disclosure;
**FIGURE 3** is a cross-sectional perspective view of an adapter for the delivery of a drug in a ventilator circuit according to one embodiment of the present disclosure;
**FIGURE 4** is a cross sectional view in the X-plane of an adapter for the delivery of a drug in a ventilator circuit according to one embodiment of the present disclosure;
**FIGURE 5** is a cross sectional view in the Y-plane of an adapter for the delivery of a drug in a ventilator circuit according to one embodiment of the present disclosure; and
**FIGURE 6** is a perspective view of the various adapters tested for the efficacy of delivering a drug in a ventilator circuit.

The Figures are not to scale and some features may be exaggerated or minimized to show details of particular elements while related elements may have been eliminated to prevent obscuring novel aspects. Therefore, specific structural and functional details disclosed herein are not to be interpreted as limiting but merely as a basis for the claims and as a representative basis for teaching one skilled in the art to variously employ the present disclosure.

### DETAILED DESCRIPTION

The terms "coupled" and "connected", along with their derivatives, may be used herein. It should be understood that these terms are not intended as synonyms for each other. Rather, in particular embodiments, "connected" may be used to indicate that two or more elements are in direct physical or electrical contact with each other. "Coupled" may be used to indicated that two or more elements are in either direct or indirect (with other intervening elements between them) physical or electrical contact with each other, or that the two or more elements co-operate or interact with each other (e.g. as in a cause and effect relationship).

The present disclosure includes an improved adapter for the delivery of a drug within or to a ventilator circuit.

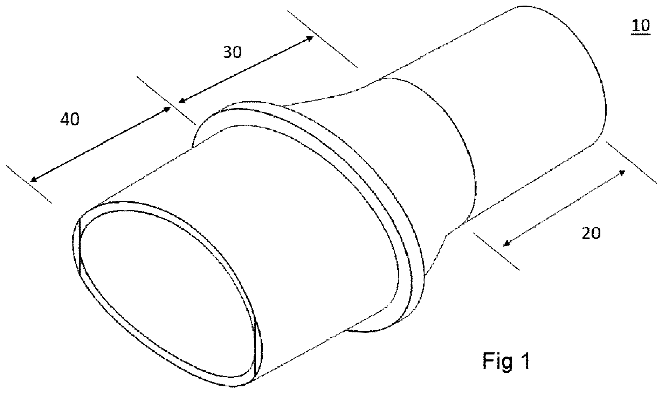

With reference to Figure 1 and according to one embodiment of the present disclosure, an improved adapter 10 for use in a ventilator circuit is shown. Adapter 10 has a ventilator section 20, a transition section 30 and a medication section 40 that allows to deposit with greater efficiency a drug within a ventilator circuit. The term drug in the present disclosure encompasses at least a mist, nebulized or aerosolized medication.

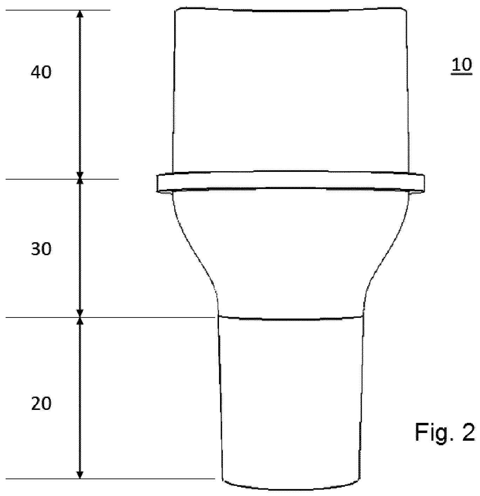

With reference to Figures 1 and 2 and according to one embodiment of the present disclosure, the ventilator section 20 is generally circular in shape and the transition section 30 has a funnel shape, whereas the medication section 40 has an oval shape. The shape of the medication section 40 can be of any shape allowing to connect a drug dispenser (not shown) to the medication section 40.

With further reference to Figure 1 and 2 and according to one embodiment of the present disclosure, the ventilator section 20 can have an internal volume within a range of 3.0 ml to 4.5 ml. Transition section 30 can have an internal volume within a range of 4.0 ml to 5.0 ml and medication section 40 can have an internal volume within a range of 7.0 ml to 9.0 ml. In a preferred embodiment, the ventilator section 20 has an internal volume of 3.7 ml, transition section 30 has an internal volume of 4.5 ml and medication section 40 has an internal volume of 7.7 ml for a total internal volume of 15.9 ml.

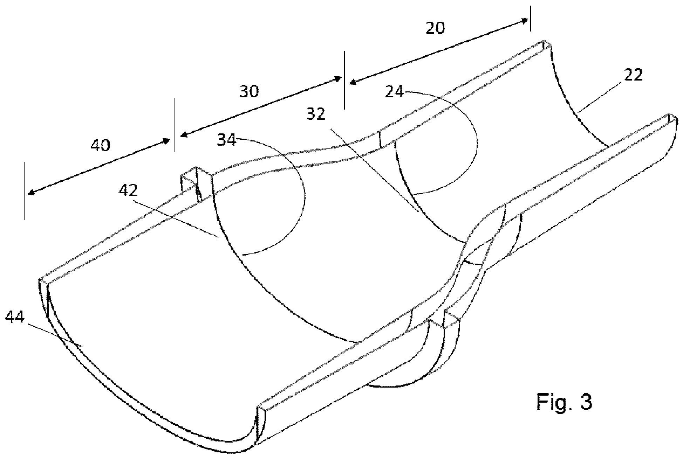

With reference to Figure 3 and according to one embodiment of the present disclosure, ventilator section 20 has a first end 22 allowing to connect the adapter 10 to a ventilator circuit (not shown) and a second end 24 to connect to the transition section 30. Meanwhile, transition section 30 has a first end 32 connected to the second end 24 of the ventilator section 20, and a second end 34 connected to the first end 42 of the medication section 40. Medication section 40 has an opposite end 44 to connect the adapter 10 to a drug dispenser (not shown).

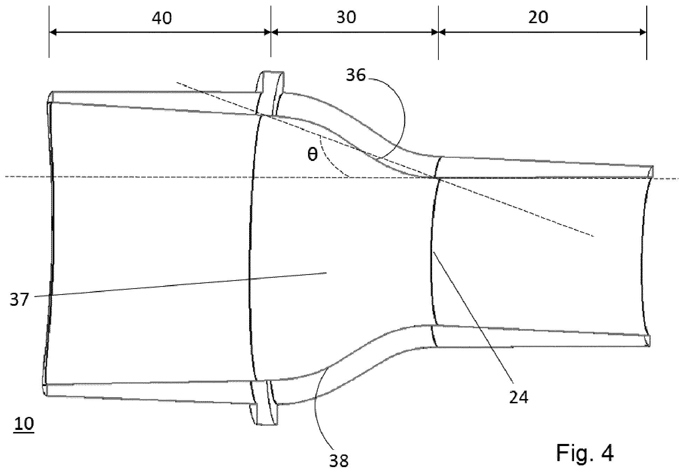

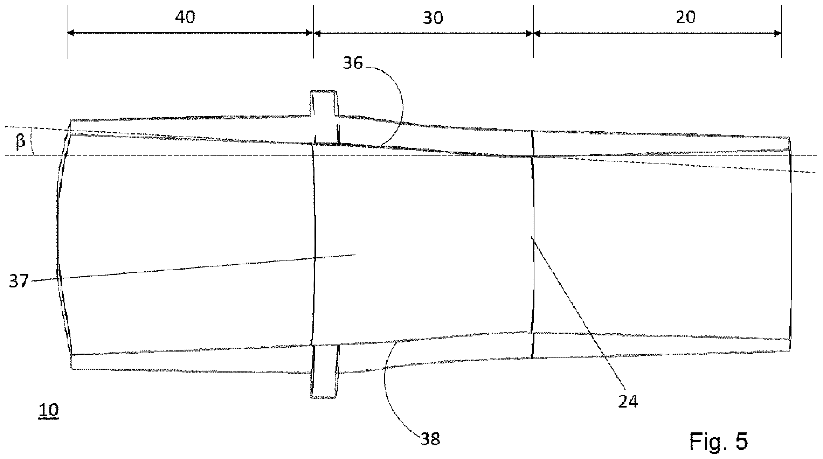

With reference to Figures 4 and 5 and according to one embodiment of the present disclosure, transition section 30 is shown having inner converging walls 36, 38, converging towards and connecting to the second end 24 of the ventilator section 20. A worker skilled in the art would appreciate that although the present disclosure describes two inner converging walls 36, 38, the adapter 10 is only comprised of a single inner converging wall 37 as the adapter 10 and the transition section 30 form a single, enclosed tubular element. Further, a worker skilled in the art would appreciate that as the transition section 30 and the adapter 10 are generally oval-shaped, a cross-section of the adapter 10 in the X-plane is different from the cross-section of the adapter 10 in the Y-plane.

With specific reference to Figure 4, the adapter 10 is shown with a cross-section in the X-plane. As shown, the converging walls 36, 38 have an angle θ with respect to the x-axis. The angle θ is defined by the equation 5 ≤ θ ≤ 89, although in a preferred embodiment θ is 21-degrees.

With specific reference to Figure 5, the adapter 10 is shown with a cross-section in the Y-plane. As shown, the converging walls 36, 38 have an angle θ with respect to the x-axis. The angle θ is defined by the equation 3 ≤ θ ≤ 6, although in a preferred embodiment θ is preferably 4.5-degrees.

With further reference to Figures 4 and 5 and according to one embodiment of the present disclosure, the inner converging wall 37 of transition section 30 is smooth and does not have any edges. The presence of edges within the transition section 30 could disrupt the travel path of a drug from a dispenser to the ventilator circuit. Indeed, the smoothness of the converging wall 37 of transition section 30, or any other internal edge of the adapter 10, directly impacts the efficacy of delivery of a drug into the ventilator circuit. The present adapter 10 can deliver a greater percentage of a drug within a ventilator circuit.

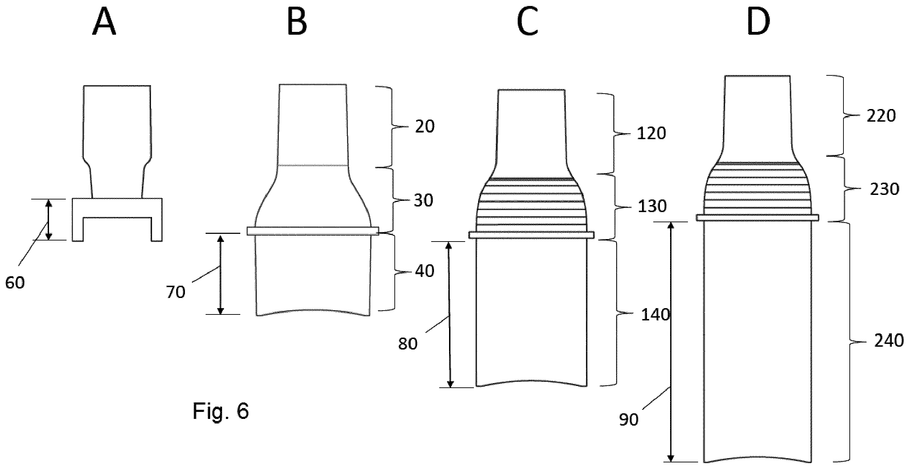

With reference to Figure 6 and according to one embodiment of the present disclosure, a testing protocol was developed for use in a simulated ventilator circuit, to demonstrate the ability of the improved adapter 10 of the present disclosure to deliver a greater percentage of drugs within a ventilator circuit. The ventilator settings for the testing were as follows:
Respiratory Rate - 12breaths/minute
Tidal Volume - 400ml
Flow rate - 45 L/minute
Inspiratory Time - 1.5 seconds
PEEP - 5 cmH₂O

The drug used in the simulated ventilator circuit was 2.5 mcg of Spiriva® Respimat® and at a dosage of 8 puffs per test into a Respirgard™ filter. Three separate tests were performed. Drug was dosed at the patient wye of the ventilator circuit just before the inspiratory breath was delivered. The test analysis consisted of first measuring the absorbance of different solutions with known concentrations to have the calibration curve for the Tiotropium. After this step, each filter (white part in the filter) was placed in 10 ml of water for 24 hours and tested the solution using the UV spectrophotometer to obtain the absorbance. A total of four different adapters were used in the testing protocol defined above. Each adapter is shown with each adapter being defined by letters A through D. Each adapter has a medication section with a specific length. The length of each medication section for each adapter tested is as follows:
Adapter A - 11 cm - medication section 60
Adapter B - 21 cm - medication section 70
Adapter C - 40 cm - medication section 80
Adapter D - 66 cm - medication section 90

The total volume of each adapter tested is as follows:
Adapter A - 5.8 ml
Adapter B - 15.9 ml
Adapter C - 23 ml
Adapter D - 44 ml

The total volume of each adapter is inclusive of the volume within the medication section based on the varying length of each medication section as well as corresponding medication and ventilator section found in each adapter tested.

Based on the above parameters, the following test results were obtained:

**TABLE 1**

| Adapter | Medication deposited in adapter | Medication delivered to patient |
|---|---|---|
| A | 0.94 ug | 2.31 ug |
| B | 0.98 ug | **2.42 ug** |
| C | 1.28 ug | 2.26 ug |
| D | 1.18 ug | 2.12 ug |

As can be seen from the test results in Table 1, adapter B allowed for the greatest amount of drug to be delivered to the patient based on the physical parameters of adapter B. According to one embodiment of the present disclosure and according to Table 1, the improved adapter is adapter B as tested in the testing protocol. The medication delivered to the patient is representative of the drug that was deposited within a filter simulating as a patient. The filter was placed at a distance that would be commonly found in use in a ventilator circuit between the adapter and the patient with the filter positioned at a distance representative of a patient.

With further reference to Table 1, the improved adapter of the present disclosure requires an internal volume that is equal or less than 15.9 ml and a medication length which cannot exceed 21 cm. The test results in Table 1 demonstrate that the delivery of medication to a patient will be less if these parameters are exceeded in an improved adapter of the present disclosure.

With further reference to Figure 6 and according to an embodiment of the present disclosure, the ventilator sections 20, 120, 220 of adapters B, C and D respectively can have an internal volume within a range of 3.0 ml to 4.5 ml. The transition sections 30, 130, 230 of adapters B, C and D respectively can have an internal volume within a range of 4.0 ml to 5.0 ml and the medication sections 40, 140, 240 of adapters B, C and D respectively can have an internal volume within a range of 7.0 ml to 34.5 ml. In the preferred embodiment that is adapter B, the ventilator section 20 has an internal volume of 3.7 ml, the transition section 30 has an internal volume of 4.5 ml and the medication section 40 has an internal volume of 7.7 ml for a total internal volume of 15.9 ml. Because of the above, the range of the adapters B, C and D ranges from 14ml to 44ml.

In another embodiment of the present disclosure, the improved adapter can be used in a non-ventilator application wherein a drug is delivered in an unassisted manner to a patient. Such applications would include, but would not be limited to, nasal, oral or tracheal as the route for delivering a drug. The improved adapter under this embodiment could be adapted to be interconnected to any device required to deliver the drug in an unassisted manner, wherein such devices could be a mask or tube.

The term ventilator section as defined in the present description can also include a connection to an unassisted application that does not include a ventilator.

## Claims

1. An improved adapter for delivering a drug, the adapter comprising:
a ventilator section for connecting to a ventilator circuit;
a transition section connected to a first end of the ventilator section, the transition section having a smooth inner converging wall to increase deposition of the drug in the ventilator circuit; and,
a medication section having one end connected to the transition section and an opposite end connected to a drug dispenser.

2. The improved adapter of Claim 1 wherein the converging wall has an angle of convergence θ in the X-plane, where 5 ≤ θ ≤ 89 and an angle of convergence θ in the Y-plane, where 3 ≤ θ ≤ 6.

3. The improved adapter of Claim 1 wherein the converging wall has an angle of convergence θ, where θ = 21.

4. The improved adapter of Claim 1 wherein the converging wall has an angle of convergence θ, where θ = 4.5.

5. The improved adapter of Claim 1 wherein an internal volume of the ventilator section is defined by X whereby: 3.0 millilitres ≤ X ≤ 4.5 millilitres.

6. The improved adapter of Claim 1 wherein an internal volume of the transition section is defined by Y whereby: 4.0 millilitres ≤ Y ≤ 5.0 millilitres.

7. The improved adapter of Claim 1 wherein an internal volume of the medication section is defined by Z whereby: 7.0 millilitres ≤ Z ≤ 34.5 millilitres.

8. The improved adapter of Claim 1 wherein the total internal volume of the adapter is within a range from 14 to 44 millilitres.

9. The improved adapter of Claim 1 wherein a total internal volume of the adapter is 15.9 millilitres.
